Europäisches Patentamt

European Patent Office

Office européen des brevets

(19)

(11) Publication number: **0 247 587**
**A2**

(12)

# EUROPEAN PATENT APPLICATION

(21) Application number: **87107693.1**

(22) Date of filing: **26.05.87**

(51) Int. Cl.⁴: **C 07 K 11/00, C 12 P 21/00**

(30) Priority: **29.05.86 JP 122204/86**

(43) Date of publication of application: **02.12.87**
**Bulletin 87/49**

(84) Designated Contracting States: **DE FR GB**

(71) Applicant: **Sanraku Incorporated, 15-1,**
**Kyobashi 1-chome Chuo-ku, Tokyo (JP)**

(72) Inventor: **Nomura, Hideo, 13-1-103, Hatori 3-chome,**
**Fujisawa-shi Kanagawa-ken (JP)**
Inventor: **Kimura, Kiyoshi, 3304 Banchi,**
**Kameino-danchi 2-104 Kameino, Fujisawa-shi**
**Kanagawa-ken (JP)**
Inventor: **Sasao, Keiichi, 99-5, Sengen-cho 2-chome**
**Nishi-ku, Yokohama-shi Kanagawa-ken (JP)**
Inventor: **Okabe, Mitsuyasu, 1-1-1074, Fujisawa**
**Seibu-danchi, 3874, Ohba, Fujisawa-shi Kanagawa-ken**
**(JP)**
Inventor: **Ishikura, Tomoyuki, 22-32, Kowada 1-chome,**
**Chigasaki-shi, Kanagawa-ken (JP)**

(74) Representative: **Patentanwälte Müller-Boré, Deufel,**
**Schön, Hertel, Lewald, Otto,**
**Postfach 26 02 47 Isartorplatz 6,**
**D-8000 München 26 (DE)**

(54) A method for enhancing the yield of depsipeptide antibiotics by fermentation.

(57) Production of depsipeptide antibiotics is improved by a
continuous or intermittent addition of glucose and/or amino
acids to the medium during cultivation.

EP 0 247 587 A2

# A METHOD FOR ENHANCING THE YIELD OF DEPSIPEPTIDE ANTIBIOTICS BY FERMENTATION

## BACKGROUND OF THE INVENTION

Depsipeptide antibiotics have broad antimicrobial spectra. They are especially highly active against Gram-positive bacteria and mycoplasmas such as Staphylococcus aureus, Streptococcus pyogenes and Diplococcus pneumoniae. Furthermore, it is well known that depsipeptide antibiotics improve feed efficiency and growth of domestic animals. Because of their biological properties, it has been reported that depsipeptide antibiotics seem to be useful antibiotics not only for therapeutics but as growth promoters of domestic animals (See Japanese Patent Kokoku No. 55-3339, Microbiological Reviews, 43, 145-198, 1979).

On the other hand, there are very few antibiotics of this group commercially available. The reason seems to be the low productivity by fermentation. To overcome this problem, screening for more suitable strains and/or fermentation conditions including medium studies has been carried out. Certain depsipeptide antibiotics productivities have been improved by the use of more suitable strains and better fermentation conditions (See Japanese Patent Kokai No. 59-59198). No satisfactory production of depsipeptide antibiotics are available.

Therefore, inventors have studied improvements of depsipeptide antibiotics production on a large scale.

Inventors disclosed that continuous or intermittent addition of glucose together with an amino acid or amino acids mixture improved the production of depsipeptide antibiotics.

Glucose is well known as a carbon catabolite represser of secondary metabolite such as antibiotics, especially during the initial phase of fermentation (See Agric. Biol. Chem., 48, 3041, 1984). By the method of this invention, glucose was effectively utilized without any catabolite repression. Furthermore, the production of depsipeptide antibiotics was increased by the addition of glucose and amino acids mixture.

## SUMMARY OF THE INVENTION

This invention relates to a new improved fermentation method for depsipeptide antibiotics by adding glucose and an amino acid or amino acids mixture to the medium during cultivation.

## DETAILED DESCRIPTION

Depsipeptide antibiotics in this invention mean peptide antibiotics having at least one amide and one ester bond. Highly modified forms of this group also belong to depsipeptide antibiotics. The examples of the former compounds are neoviridogriseins group, viridogrisein, etamycin, mikamycins B, vernamycins B group, ostreogrycins B group, streptogramin B, virginiamycins S group and their related antibiotics.

And the examples of the latter compounds are griseoviridin, mikamycin A, ostreogrycins G group, madumycin II, pristomycin IIA, streptogramin A and their related antibiotics. (e. g. see the above literature and Microbiological Reviews, 43 145 to 198, 1979).

The fermentation production methods of these antibiotics are as follows:

Production of neoviridogrisein by Streptomyces Sp. P8648 (FERM P-3562) (See Japanese Patent Kokoku No. 55-3339),

production of virginiamycins group by Streptomyces virginiae (ATCC 13161) (See Antibiot. and Chemother., 3, 1283, 1953),

production of mikamycins group by Stretomyces mitakaensis (See J. Antibiot., IIA, 14, 1958),

production of vernamycins group by Streptomyces loidensis (See U.S.Pat. No. 2990326), and

production of streptogramins by Streptomyces graminofaciens (See Antibiot. and Chemother., 3, 1283, 1953).

As the additive amount of glucose and amino acids vary depending upon the medium and/or the producing organism, it is not critical. However, generally speaking, glucose can be added and adjusted to the concentration of about 1 to 5% (w/w) together with 0.1 to 1% (w/w) of amino acids. Any free form of amino acids and any substances containing amino acids can be employed. For example, it is preferable that as free amino acids are glutamic acid, lysine, methionine, etc. and as substances containing amino acids are casein hydrolysate, soybean hydrolysate, corn-steep liquor, casein, casamino acid solution, etc., they are favorably used as substances having a great deal of amino acids.

Glucose and amino acids or substance containing amino acids are separately sterilized. They can be separately fed to the culture or after combining both solutions to a suitable concentration, the mixture can be fed to the culture. Feeding is carried out by the use of pumps or by stationary pressure. During the cultivation, it is desirable that the pH continuously maintained between 6.5 and 7.5 by a pH sensor.

A wide variety of nutrient media well known and employed for cultivation of microorganisms belonging to stretomyces may be used as the media for depsipeptide antibiotics production. These and any other compounds can be employed for production as long as they do not exhibit any inhibition of depsipeptide antibiotics production. For example, the following compounds can be used as carbon sources: glucose, glycerol, starch, dextrin, oat meal, beet molassess, oil, fat, etc. The following compounds can be used as nitrogen sources: soybean meal, cotton seed meal, meat extract, peptone, dry yeast, corn-steep liquor, yeast extract, casein, casein hydrolysate, etc. It is necessary that glucose, corn-steep liquor and casein should be used below certain concentration at which they will inhibit production of depsipeptide antibiotics.

If necessary, the medium may also contain minerals commonly used in nutrient media. Some specific samples of such minerals constituents are calcium carbonate, sodium chloride, potassium chloride, phosphates, magnesium sulfate and other trace metal salt. Furthermore, some vitamins and organic acids can be added to the medium.

The preferred fermentation method for depsipeptide antibiotics production involves liquid fermentation method such as shaking culture method or tank culture method. It is desirable that fermentation carried out under aerobic conditions between 25°C to 33°C for 2 to 10 days. In this case, during fermentation, preferably the pH of the culture is controlled and maintained between 6 to 9, and especially adjusted between 6.5 to 8.5 at the start of cultivation.

Depsipeptide antibiotics accumulated in the fermentation broth can be recovered by any well known recovering procedure. For example, the antibiotics can be extracted by organic solvent such as ethyl acetate, butyl acetate, n-butyl alcohol and chloroform, and if necessary, the antibiotics can be treated with some positive and negative ion-exchange resin and/or absorption resin.

By the method disclosed in this invention, productivity of depsipeptide antibiotics is greatly improved, compared with any conventional production. The following description shows one production methods disclosed in this invention to be explained in further detail.

EXAMPLE 1    Production of neoviridogrisein by fed batch culture using a mini jar fermentor

150 milliliters of seed culture medium consisting of 3% corn starch, 2% soybean meal, 0.2% glucose, 0.2% yeast extract, 0.1% NaCl, 0.1% $K_2HPO_4$, 0.05% $MgSO_4 \cdot 7H_2O$, were poured into a 500-ml Erlenmeyer flask which is autoclaved at 121°C for 20 minutes. After autoclaving, a loopful amount of Streptomyces Sp. G-89 (FERM BP-184) spores was inoculated and the flask was incubated at 28°C for 48 hours under a rotary shaking (240 rpm).

1 liter sterile medium consisting of 4% glucose, 2% soybean meal, 0.5% casamino acid, 0.05% $MgSO_4 \cdot 7H_2O$, 0.1% $MnCl_2 \cdot 4H_2O$, 0.01% $ZnSO_4 \cdot 7H_2O$ was poured into a sterile 2.5-liter mini jar fermentor. After 20 ml of the seed culture described previously were added to the fermentor. The incubation was carried out under the following conditions: aeration, 0.5vvm; agitation, 400 rpm; temperature, 28°C. From 48 hours after inoculation, feed solution was added to the fermentor at the rate of 50ml/day/fermentor. The feed solution was composed of 40% glucose and 48% casamino acid. Since the pH of the culture was down, the culture was automatically controlled to pH 6.7 by adding ammonia water.

- 6 -

The production of neoviridogrisein was greatly increased by this method. The medium volume and the accumulation of neoviridogrisein during cultivation by this method are shown in Table 1.

Table 1

| Cultivation time (day) | 3 | 4 | 5 | 6 | 7 | 8 | 9 | 10 | 11 | 12 |
|---|---|---|---|---|---|---|---|---|---|---|
| Medium volume (ml) | 1070 | 1100 | 1150 | 1200 | 1260 | 1330 | 1400 | 1430 | 1520 | 1600 |
| Accumulation as neoviridogriseins* (µg/ml) | 190 | 320 | 500 | 620 | 810 | 1000 | 1100 | 1200 | 1220 | 1250 |
| Total amount (g) | 0.20 | 0.35 | 0.58 | 0.74 | 1.02 | 1.33 | 1.46 | 1.71 | 1.85 | 2.00 |

\*   Quantitative determination by thin layer chromatography: 2 ml of the cultured broth were diluted with buffer solution (pH 5.75) and the antibiotics were extracted with 4ml of benzene/ethyl-acetate mixture (1:1). The suitable amount of the extract was loaded onto a precoated silica-gel plate Art. No. 5715 (E. Merck Co. product) and the plate was developed by $CHCl_3$/MeOH/pyridine (130:4:0.25). After development, neoviridogriseins were measured by fluorometry. The quantitative analyses were carried out by use of chromatoscanner (Model CS-920, Shimadzu Co. product). The wavelength of excitation was 365nm with a No.2 interference filter.

0 247 587

EXAMPLE 2  Production of neoviridogriseins by fed
batch culture using a 30-liter jar
fermentor

200 milliliters of the seed culture of
Stretomyces SP G-89 described in Example 1 were
inoculated into a 20-liter jar fermentor (Marubishi
Co. product) containing 10 liters of the same seed
medium as that used in Example 1. Cultivation was
carried out at 28°C for 48 hours under aeration of
0.5vvm and under agitation (350 rpm) after said seed
culture was sterilized at 120°C for 15 minutes.
Seed culture prepared in the same manner described
in Example 1 was added to the seed culture by 200
milliliters.  15 liters of the production medium
consisting of 8% corn starch, 2% glucose, 3% soybean
meal, 0.25% casamino acid, 0.05% yeast extract, 0.1%
NaCl, 0.1% $K_2HPO_4$, 0.1% $MnCl_2$ $4H_2O$, 0.05% $MgSO_4$ $7H_2O$,
0.01% $ZnSO_4$ $7H_2O$, 0.02% Spitase M, 0.02% PN-4, and
18 milliliters of antifoam (Adekanol) was poured in
a 30-liter jar fermentor (Marubishi Co. product) and
liquefied for 10 minutes at 70°C. After that, the
medium was sterilized at 121°C for 20 minutes.
Then, 500 milliliters of the seed culture was added
and cultivated at 28°C under aeration of 0.5vvm and
agitation, 300 rpm.  From 66 hours after
inoculation, feed solution consisting of 15%
casamino acid was continuously fed at the rate of
400 ml per day per fermentor. From 114 hours after said inoculation, 40% glucose was continuously fed at the rate of 400 ml per day per fermentor. The production of neoviridogriseins was greatly improved by the method of this invention. The results are shown in Table 2.

Table 2

| Cultivation time (day) | 4 | 5 | 6 | 7 | 8 | 9 | 10 | 11 | 12 |
|---|---|---|---|---|---|---|---|---|---|
| Medium volume (l) | 15.45 | 16.35 | 17.55 | 18.15 | 19.35 | 21.00 | 22.35 | 23.7 | 24.9 |
| Accumulation as neoviridogriseins* ($\mu$g/ml) | — | 224 | 387 | 737 | 824 | 913 | 990 | 1408 | 1652 |
| Total amount (g) | — | 3.66 | 6.79 | 13.30 | 15.94 | 19.17 | 22.12 | 33.3 | 41.1 |

*   Quantitative determination method of neoviridogriseins was the same as that in Example 1.

0 247 587

EXAMPLE 3   Production of viridogriseins in a mini

jar fermentor by fed batch culture

One loopful of Stretomyces Sp. P8648 (FERM P-3562) was inoculated to a 500-ml Erlenmeyer flask containing 100 ml of the same seed medium described in Example 1, and cultivation was carried out at 28°C for 48 hours under rotary shaking of 240 rpm. The seed culture was poured into a 2.5-liter mini jar fermentor containing 1 liter of the following sterile fermentation medium: 4% glucose, 3% soybean meal, 0.2% sodium glutamate, 0.1% $K_2HPO_4$, 0.05% $MgSO_4$ $7H_2O$ and 0.1% antifoam (Adekanol LG-121, Asahi Denka Co.). The cultivation was carried out at 28°C under aeration of 0.5vvm and under agitation of 500 rpm. From 72 hours after inoculation, 15 ml of 15% casamino acid solution which was prepared separately was added to the fermentor once a day. During the fermentation, pH of the culture was controlled to about 7 by adding glucose. The pH was automatically controlled by the use of a pH sensor together with a pump to supply glucose.

The accumulation amount of viridogrisein was improved by this fed batch culture. The results are shown in Table 3.

Table 3

| Cultivation time (Day) | 3 | 4 | 5 | 6 | 7 | 8 | 9 | 10 | 11 | 12 | 13 |
|---|---|---|---|---|---|---|---|---|---|---|---|
| Medium volume (ml) | 1050 | 1120 | 1150 | 1210 | 1250 | 1330 | 1390 | 1420 | 1500 | 1530 | 1590 |
| Accumulation as neoviridogrise -ins* (µg/ml) | 64 | 213 | 243 | 394 | 532 | 710 | 890 | 1120 | 1300 | 1460 | 1500 |
| | 0.06 | 0.23 | 0.28 | 0.48 | 0.66 | 0.94 | 1.23 | 1.60 | 1.95 | 2.23 | 2.38 |
| Total amount (g) | | | | | | | | | | | |

\* Quantitative analysis was accomplished by the same method described in Example 1.

Reference Example 1:    Production of neoviridogriseins in a mini jar fermentor by batch culture

    1 liter of the fermentation medium consisting
of 10% corn starch, 5% soybean meal, 0.1% NaCl, 0.1%
$K_2HPO_4$, 0.1% $MnCl_2 \cdot 7H_2O$, 0.05% $MgSO_4 \cdot 7H_2O$, 0.1%
$ZnSO_4 \cdot 7H_2O$ and 0.1% silicon was liquefied by 10 mg
(0.01% based of starch) of α-amylase (Spitase PN-4,
Nagase Sangyo Co.) in an Erlenmeyer at 80°C for 15
minutes.    After liquefaction, the medium was
autoclaved at 121°C for 10 minutes.    The sterile
medium was poured into a mini jar fermentor
sterilized with an autoclave at 121°C for 20
minutes.    50 ml of the seed culture prepared by the
method described in the Example 1 were added to the
fermentor.    Cultivation was conducted at 28°C under
aeration of 0.5vvm and agitation of 400 rpm.    In
this case, no compound was added to the fermentor
during the cultivation.    The results are shown in
Table 4.

Table 4

| Cultivation time (Day) | 2 | 3 | 4 | 5 | 6 | 7 | 8 | 9 | 10 | 11 |
|---|---|---|---|---|---|---|---|---|---|---|
| Accumulation as neoviridogriseins* ($\mu$g/ml) | — | 45 | 185 | 354 | 580 | 820 | 1016 | 1280 | 1595 | 1656 |

\* Quantitative analysis of the production of viridogrisein was determined by the same method described in Example 1.

0 247 587

A liquid volume of culture medium after cultivated for 11 days was 800 ml and total amount of neoviridogriseins was 1.32 g.

Reference Example 2:     <u>Production of neoviridogriseins in a 30-liter jar fermentor by batch fermentation</u>

15 liters of the production medium consisting of 8.0% corn starch, 2.0% glucose, 4.5% soybean meal, 0.5% casamino acid, 0.05% yeast extract, 0.1% NaCl, 0.1% $K_2HPO_4$, 0.1% $MnCl_2 \cdot 4H_2O$, 0.05% $MgSO_4 \cdot 7H_2O$, 0.01% $ZnSO_4$ $7H_2O$, 0.015% Spitase M, 0.015% Spitase PN-4, 18 ml of Adekanol were liquefied at 70°C for 10 minutes in a 30-liter jar fermentor. After liquefaction, the fermentor was sterilized at 121°C for 20 minutes. 500 milliliters of the seed culture described in Example 1 were poured into the fermentor. Cultivation was carried out at 28°C under the agitation of 400 rpm and the aeration of 0.5 vvm. Time course of the production of neoviridogrisein is shown in Table 5 .

Table 5

Table 5

| Cultivation time (Day) | 3 | 4 | 5 | 6 | 7 | 8 | 9 | 10 | 11 |
|---|---|---|---|---|---|---|---|---|---|
| Accumulation as neoviridogriseins* (µg/ml) | 51 | 219 | 444 | 512 | 802 | 905 | 1055 | 1282 | 1458 |

* Quantitative analysis of the production of viridogrisein was determined by the same method described in Example 1.

- 17 -

A liquid volume of culture medium after cultivated for 11 days was 15 1, a yield of total neoviridogriseins was 21.8 grams.

As shown in Tables 1 to 5, it is very clear that the fed batch cultivation method disclosed in this invention for producing depsipeptide antibiotics brings a remarkable production increase effect in comparison with any conventional production methods showing the highest production level.

For Example, in the case of neoviridogriseins production, 41.4g of the antibiotics was produced in 30-1 fermentor by the method of this invention while only 17.5 g of the antibiotics was accumulated by the usual fermentation method. The reason why the cultivation time (days) varies from one to the other is that cultivation under usual fermentation methods can not be continued is because antibiotics produced by the latter fermentation method may decompose thereby causing the production itself to be reduced if it is cultivated for more than the number of days described in the reference examples.

WHAT IS CLAIMED IS:

1. A method for enhancing the yield of depsipeptide antibiotics by fermentation which comprises feeding glucose and amino acids intermittently or continuously into the nutrient medium during the cultivation.

2. The method claimed in Claim 1, wherein depsipeptide antibiotics thus produced belong to one of selected from the group consisting of neoviridogrisein, viridogrisein, eytamycin, mikamycin, vernamycin, ostreogrycin, streptogramin and virginiamycin.